# EUROPÄISCHE PATENTSCHRIFT

(11) **EP 1 703 936 B1**
(45) Veröffentlichungstag und Bekanntmachung des Hinweises auf die Patenterteilung: **24.02.2010**
(21) Anmeldenummer: 05706668.0
(22) Anmeldetag: 12.01.2005
(51) Int. Cl.: A61M 25/00

(54) **VORRICHTUNG ZUR BEHANDLUNG VON HARNBLASEN-ENTLEERUNGSSTÖRUNGEN EINES MENSCHEN**
DEVICE FOR TREATING BLADDER EMPTYING DYSFUNCTIONS OF A HUMAN
DISPOSITIF POUR TRAITER DES TROUBLES DE VIDAGE DE LA VESSIE CHEZ UN ETRE HUMAIN

(30) Priorität: 12.01.2004 DE 102004001670
(43) Veröffentlichungstag der Anmeldung: 27.09.2006
(73) Patentinhaber: Medi-Globe GmbH, 83101 Rohrdorf-Achenmühle (DE)
(72) Erfinder: MRONCZ, Andreas, 71065 Sindelfingen (DE); WOHNHAS, Gabriele, 97493 Bergrheinfeld (DE)
(74) Vertreter: Nätebusch, Roderich
(86) Internationale Anmeldenummer: PCT/DE2005/000029
(87) Internationale Veröffentlichungsnummer: WO 2005/065758

(56) Entgegenhaltungen:
- DE-A1- 19 621 420
- US-A- 4 249 536

## Beschreibung

Die Erfindung bezieht sich auf eine Vorrichtung zur Behandlung von Harnblasen-Entleerungsstorungen eines Menschen gemäß dem Oberbegriff von Anspruch 1.

Eine Vorrichtung der vorstehend genannten Art ist bereits bekannt (DE 196 21 420 C2). Es hat sich nun in der Praxis herausgestellt, dass bei der betreffenden bekannten Vorrichtung ein relativ hoher mechanischer Druck ausgeübt werden muss, um das am proximalen Vorrichtungsende vorgesehene selbstschließende Ventil zu öffnen. Die Ausübung eines derartigen hohen mechanischen Drucks wird jedoch vom Patienten, in welchem die betreffende Vorrichtung insgesamt eingesetzt ist, als unangenehm empfunden mit der Folge, dass die Akzeptanz des Einsatzzes der betreffenden bekannten Vorrichtung nicht besonders groß ist.

Der Erfindung liegt daher die Aufgabe zugrunde, eine Vorrichtung der eingangs genannten Art so weiterzubilden, dass das am proximalen Vorrichtungsende vorgesehene Ventil mit einem geringeren mechanischen Betätigungsdruck als bei der bekannten Vorrichtung geöffnet werden kann, so dass die Akzeptanz des Einsatzes der so weiter entwickelten Vorrichtung gesteigert werden kann.

Gelöst wird die vorstehend aufgezeigte Aufgabe bei einer Vorrichtung der eingangs genannten Art erfindungsgemäß durch die im kennzeichnenden Teil des Patentanspruchs 1 angegebenen Merkmale.

Die Erfindung zeichnet sich durch den Vorteil eines konstruktiv relativ einfach aufgebauten Betätigungsmechanismus für das genannte Ventil aus und kommt dabei mit einem relativ geringen mechanischen Druck aus, um das am proximalen Endabschnitt des Katheters vorgesehene Verschlussteil für einen ungehinderten Harndurchtritt zu öffnen. Das bei der vorliegenden Erfindung hierzu genutzte Konstruktionsprinzip zum Öffnen und Schließen des erwähnten Ventils ist wesentlich einfacher als das bei der oben erwähnten bekannten Vorrichtung vorgeschlagene Konstruktionsprinzip zum Ventilöffnen und -schließen, gemäß dem unter anderem ein Betätigungsmechanismus mit einem biegeelastischen Federstab verwendet wird. Damit dürfte der Einsatz der Vorrichtung gemäß der Erfindung eine bessere Akzeptanz finden als die bisher bekannte Vorrichtung.

Vorzugsweise gestattet das Verschlussteil mit einer konisch ausgebildeten Anlagefläche den Harn-Entleerungskanal des Katheters an einer Abschlusskante am proximalen Katheterende abzudichten. Diese Maßnahme bringt den Vorteil einer besonders wirksamen Abdichtung des Harn-Entleerungskanals mit sich.

Zweckmäßigerweise sind das Verschlussteil und der proximale Endabschnitt des Katheters mittels Halteelementen elastisch miteinander verbunden. Dies bringt den Vorteil einer besonders einfachen Gestaltung des Verschlussteiles und des Katheters mit sich.

Gemäß einer anderen zweckmäßigen Weiterbildung der vorliegenden Erfindung ist das Verschlussteil mit dem proximalen Endabschnitt des Katheters durch ein Gelenkelement sowie mittels wenigstens eines elastischen Halteelements verbunden. Durch die Verwendung des erwähnten Gelenkelements ist dabei auf einfache Weise eine gesicherte Positionierung des Verschlussteiles an dem Katheter ermöglicht.

Eine konstruktiv besonders einfache Möglichkeit der Verbindung des Verschlussteiles und des proximalen Endabschnitts des Katheters mittels der Halteelemente ergibt sich gemäß weiterer zweckmäßiger Ausgestaltung der Erfindung dadurch, dass das jeweilige elastische Halteelement an dem Verschlussteil oder am proximalen Endabschnitt des Katheters gebildet und mit seinem jeweils anderen Ende am proximalen Ende des Katheters bzw. am Verschlussteil gesondert befestigt ist.

Eine besonders einfache Befestigung des erwähnten jeweils anderen Endes des jeweiligen Halteelements ergibt sich in zweckmäßiger Weise dadurch, dass es mit dem betreffenden anderen Ende am proximalen Ende des Katheters bzw. Verschlussteils durch eine Klebverbindung befestigt ist.

Von ganz besonderem Vorteil ist es, wenn gemäß weiterer zweckmäßiger Ausgestaltung der vorliegenden Erfindung der mit dem Betätigungsballon verbundene Verbindungskanal im Bereich des proximalen Endabschnitts des Katheters ein durch ein gesondertes Ventil abgetrenntes Drucklumen enthält, auf dessen Füllen mit dem Betätigungsfluid durch Betätigen des genannten Betätigungsballons das Verschlussteil aus dessen Dichtungsanlage an dem genannten Harn-Entleerungskanal des Katheters abhebbar ist. Das betreffende Drucklumen wird durch Betätigen des Betätigungsballons gewissermaßen mit Betätigungsfluid aufgepumpt, ähnlich dem Aufpumpen beispielsweise eines Fahrradschlauchs. Es findet hier also gewissermaßen eine hydraulische Druckverstärkung statt. Dadurch kommt man mit einer besonders geringen mechanischen Betätigungskraft im Bereich des Betätigungsballons aus, um dennoch einen zum Öffnen des erwähnten Verschlussteiles führenden hydraulischen Druck des Betätigungsfluids in dem erwähnten abgetrennten Drucklumen bereitzustellen.

Um bei der zuvor betrachteten zweckmäßigen Weiterbildung das Verschlussteil lediglich während einer begrenzten Zeitspanne zu öffnen, ist das genannte Ventil gemäß weiterer zweckmäßiger Ausgestaltung der vorliegenden Erfindung so ausgebildet, dass es ein Einströmen des Betätigungsfluids in das genannte Drucklumen mit einer ersten, relativ hohen Geschwindigkeit ermöglicht und eine Rückströmung des betreffenden Betätigungsfluids aus dem genannten Drucklumen mit einer demgegenüber wesentlich geringeren zweiten Geschwindigkeit zulässt. Hierdurch ergibt sich in vorteilhafter Weise ein besonders geringer konstruktiver Aufwand, um das zeitlich begrenzte Öffnen des erwähnten Verschlussteiles zu erreichen.

Von Vorteil bei der zuletzt betrachteten zweckmäßigen Weiterbildung der vorliegenden Erfindung ist es, wenn das das Drucklumen von dem Betätigungsballon trennende genannte Ventil einen Ventilschaft aufweist, der im Drucklumen eine vom Betätigungsballon her zugängliche Durchgangsöffnung aufweist, die von einem Ventilschlauch umgeben ist. Im Prinzip entspricht ein derartiges Ventil einem bekannten Fahrradschlauchventil. Zur zeitlich begrenzten Öffnung des genannten Verschlussteiles lässt sich dabei der Effekt ausnutzen, dass nach dem "Aufpumpen" des erwähnten Drucklumens das in diesem befindliche Betätigungsfluid versucht, zwischen dem Ventilschaft und dem Ventilschlauch zu entweichen und eine Entlastung in dem betreffenden Drucklumen herbeizuführen. In diesem Zusammenhang hat sich gezeigt, dass die Länge, mit der der Ventilschlauch über die erwähnte Durchgangsöffnung des Ventilschaftes hinausragt, maßgebend für die Verweildauer des Betätigungsfluids in dem genannten Drucklumen und damit für die Öffnungsdauer des erwähnten Verschlussteiles ist. Je kürzer dieser Überstand ist, umso kürzer ist die Öffnungszeit des erwähnten Verschlussteiles.

Vorzugsweise bestehen der Katheter und der bzw. das Verschlussteil aus Silikon, und zweckmäßigerweise bestehen auch die Ballonanordnung und der Betätigungsballon aus Silikon. Damit wird für die betreffenden Elemente jeweils ein biokompatibles Material verwendet.

Als Betätigungsfluid wird in vorteilhafter Weise bei der vorliegenden Erfindung Öl verwendet. In diesem Zusammenhang hat sich der Einsatz von Olivenöl besonders bewährt, da dieses Öl überhaupt keine Probleme in der Anwendung mit sich bringt. Olivenöl, so hat sich gezeigt, besitzt nämlich keine Neigung, durch das jeweilige Material, und insbesondere durch Silikon des Katheters, des Verschlussteiles, der Ballonanordnung und des Betätigungsballons hindurch zu diffundieren. Der Grund hierfür dürfte nach derzeitiger Erkenntnis darin liegen, dass die Olivenölmoleküle größer sind als Moleküle anderer Öle und deshalb nicht durch die Molekülstruktur des jeweils verwendeten Materials, insbesondere des Silikons hindurchdiffundieren können.

Anhand von Zeichnungen wird die Erfindung an Ausführungsbeispielen näher erläutert.
- Fig. 1: zeigt in einer vergrößerten schematischen Darstellung eine Ausführungsform einer Vorrichtung gemäß der vorliegenden Erfindung.
- Fig. 2: zeigt eine vergrößerte Schnittansicht gemäß der in Fig. 1 eingetragenen Schnittlinie II-II.
- Fig. 3: zeigt in einer vergrößerten Schnittansicht eine in Fig. 1 mit A bezeichnete Einzelheit.
- Fig. 4: zeigt die in Fig. 3 dargestellte Einzelheit A in einer anderen Schnittebene.
- Fig. 5: zeigt in einer vergrößerten Schnittansicht eine alternative Realisierung der in Fig. 3 dargestellten Einzelheit A.
- Fig. 6: zeigt ausgehend von der in Fig. 3 dargestellten Schnittansicht zum einen die Verhältnisse, die sich bei Wirksamsein eines Betätigungsfluids ergeben, sowie die Ausbildung eines gesonderten Drucklumens in einem Betätigungskanal.
- Fig. 7: veranschaulicht in einer vergrößerten Schnittansicht eine in Fig. 1 mit B bezeichnete Einzelheit.
- Fig. 8: zeigt eine vergrößerte Schnittansicht entsprechend der in Fig. 1 eingetragenen Schnittlinie VIII-VIII.
- Fig. 9: veranschaulicht in einer vergrößerten Schnittansicht eine in Fig. 1 mit C bezeichnete Einzelheit C.
- Fig. 10: veranschaulicht in einer vergrößerten Schnittansicht eine in Fig. 6 mit D bezeichnete Einzelheit.
- Fig. 11: veranschaulicht in einer vergrößerten Schnittansicht eine alternative Realisierungsform der in Fig. 11 dargestellten Einzelheit.

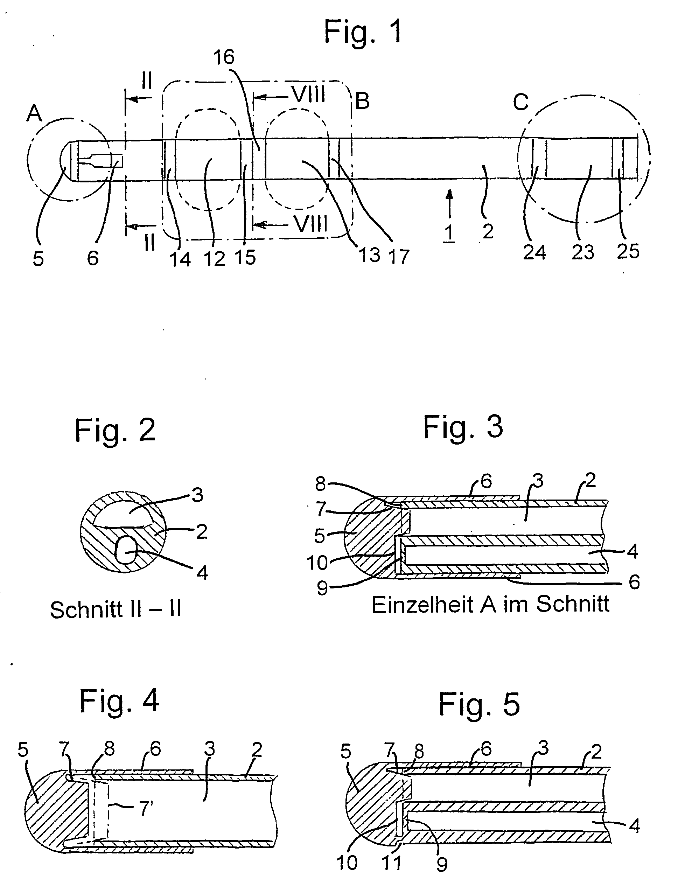

In Fig. 1 ist in einer vergrößerten schematischen Darstellung eine Ausführungsform einer Vorrichtung 1 gemäß der vorliegenden Erfindung dargestellt. Die betreffende Vorrichtung 1 weist einen Katheter 2 auf, der, wie nachstehend noch näher ersichtlich werden wird, unter anderem einen Harn-Entleerungskanal aufweist und der an seinem in Fig. 1 links dargestellten proximalen Endabschnitt ein Ventil trägt, mit dessen Hilfe der betreffende Harn-Entleerungskanal geöffnet und geschlossen werden kann. An dieser Stelle sei angemerkt, dass bei Anwendung der Vorrichtung 1 der erwähnte proximale Endabschnitt des Katheters 2 in der Harnblase eines Menschen liegt, in dessen Harnröhre der betreffende Katheter 2 untergebracht ist. Vorzugsweise bestehen der Katheter und das erwähnte Ventil aus biokompatiblem Silikon.

Der in Fig. 1 links dargestellte proximale Endabschnitt des Katheters 2 weist einen Querschnitt auf, wie er beispielsweise in Fig. 2 dargestellt ist, die eine vergrößerte Schnittansicht längs der in Fig. 1 eingetragenen Schnittlinie II-II zeigt. Wie aus Fig. 2 hervorgeht, enthält der Katheter 2 in dem betreffenden proximalen Endabschnitt den erwähnten Harn-Entleerungskanal 3 und einen weiteren, hier auch als Verbindungskanal bezeichneten Betätigungskanal 4 auf, in welchem ein Betätigungsfluid zur Wirkung gebracht wird, wie dies weiter unten noch näher ausgeführt wird. Als Betätigungsfluid wird hier Öl, vorzugsweise Olivenöl verwendet. Der Harn-Entleerungskanal 3 und der Betätigungskanal 4 sind hier zwar mit ovalförmigen Querschnitten dargestellt; es dürfte jedoch einzusehen sein, dass sie auch andere Querschnitte besitzen könnten.

Das an dem erwähnten proximalen Endabschnitt des Katheters 2 vorgesehene Ventil umfasst ein in Fig. 1 mit 5 bezeichnetes Verschlussteil. Dieses Verschlussteil 5, dessen Aufgabe darin besteht, den Harn-Entleerungskanal 3 an dem betreffenden proximalen Endabschnitt des Katheters 2 abzudichten und bei Bedarf für einen ungehinderten Harndurchtritt zu öffnen, ist gemäß Fig. 1 mit dem proximalen Ende des Katheters 2 mittels eines lappenförmig dargestellten Halteelements 6 elastisch verbunden. An dieser Stelle sei angemerkt, dass von dem Halteelement 6 eine vorgegebene Anzahl, beispielsweise drei oder vier Halteelemente vorgesehen sein können. Das jeweilige Halteelement 6 kann dabei lediglich mit seinem in Fig. 1 dargestellten breiten Lappenteil mit dem proximalen Ende des Katheters 2 verbunden sein. Der dünne Stegteil des jeweiligen Halteelements 6 verleiht diesem die Elastizität zum Schließen und Öffnen des das Verschlussteil 5 enthaltenden Ventils.

In Fig. 3 ist die in dem in Fig. 1 mit A bezeichneten Bereich vorhandene Einzelheit A in einer vergrößerten Schnittansicht dargestellt. Wie ersichtlich, sind in dem Katheter 2 der Harn-Entleerungskanal 3 und der Betätigungskanal 4 vorhanden. Der Harn-Entleerungskanal 3 wird von dem Verschlussteil 5 mittels eines eine konisch ausgebildete Anlagefläche aufweisenden Anlageteiles 7 dadurch abgedichtet, dass die betreffende konisch ausgebildete Anlagefläche an einer proximalen Endkante 8 des den Harn-Entleerungskanal 3 umgebenden Kathetermaterials anliegt. Eine derartige Dichtung ist dabei wirksamer als eine flächenmäßige Abdichtung zwischen der Anlagefläche 7 und einer entsprechend ausgebildeten Anlagefläche am proximalen Ende des Harn-Entleerungskanals 3.

Das Verschlussteil 5 ist gemäß Fig. 3 mit dem proximalen Endabschnitt des Katheters 2 durch zwei Halteelemente 6 verbunden, die vorzugsweise mit der Außenseite des proximalen Endabschnitts des Katheters 2 in der im Zusammenhang mit Fig. 1 erläuterten Weise verbunden sind, und zwar durch eine Klebverbindung. Für diese Klebverbindung kommt ein sogenannter medizinischer Kleber in Frage, der biokompatibel ist.

Aus Fig. 3 ist ferner ersichtlich, dass der Betätigungskanal 4 an seinem proximalen Ende durch eine Verschlusswand 9 verschlossen ist, die hier als von relativ geringer Dicke dargestellt ist. Diese Verschlusswand 9 liegt einer entsprechend geformten Betätigungsfläche 10 des Verschlussteiles 5 gegenüber. In Fig. 3 ist zwar zwischen der Verschlusswand 9 und der Betätigungsfläche 10 ein Zwischenraum dargestellt; in der Praxis muss dieser Zwischenraum jedoch nicht vorgesehen sein.

Fig. 4 veranschaulicht die in Fig. 3 gezeigte Einzelheit A bei Betrachtung lediglich des Harn-Entleerungskanals 3 im Schnitt. Während Fig. 3 die Verhältnisse veranschaulicht, die sich aus einer Betrachtung in vertikaler Schnittrichtung bei der Schnittansicht gemäß Fig. 2 ergeben, zeigt Fig. 4 die Verhältnisse, die gemäß Fig. 2 in horizontaler Schnittrichtung durch den Harn-Entleerungskanal 3 vorliegen. Gemäß Fig. 4 ist das Verschlussteil 5 mit seinem konisch ausgebildeten Anlageteil 7 von der proximalen End- bzw. Abschlusskante 8 des Harn-Entleerungskanals 3 soweit weggeführt, dass der Harn-Entleerungskanal 3 des Katheters 2 für einen ungehinderten Harndurchtritt geöffnet ist. In diesem Falle sind die in Fig. 4 dargestellten Halteelemente 6, die an dem Katheter 2 befestigt sind, elastisch gedehnt. Sobald diese elastische Dehnung aufgehoben ist, kehrt das Verschlussteil 5 mit seinem konisch ausgebildeten Anlageteil 7 in die in Fig. 4 mit 7' bezeichnete Stellung zurück, in der der proximale Endabschnitt des Harn-Entleerungskanals 3 dicht verschlossen ist.

Fig. 5 zeigt in einer vergrößerten Schnittansicht ähnlich der in Fig. 3 dargestellten Schnittansicht eine alternative Form der Verbindung des Verschlussteiles 5 mit dem proximalen Endabschnitt des Katheters 2. Gemäß Fig. 5 wird der Harn-Entleerungskanal 3 des Katheters 2 wie in Fig. 3 dadurch verschlossen, dass das konisch ausgebildete Anlageteil 7 des Verschlussteiles 5 an der proximalen End- bzw. Abschlusskante 8 des betreffenden Harn-Entleerungskanals 3 dichtend anliegt. Der Betätigungskanal 4 ist im gleichen Zustand dargestellt wie in Fig. 3, wobei der proximale Endabschnitt bzw. die Verschlusswand 9 des Betätigungskanals 4 wie in Fig. 3 der Betätigungsfläche 10 des Verschlussteiles 5 gegenüberliegt. Im Unterschied zu den in Fig. 3 dargestellten Verhältnissen ist jedoch gemäß Fig. 5 das Verschlussteil 5 mittels eines Gelenkelements 11 mit dem Katheter 2 und mittels wenigstens eines elastischen Halteelements 6 verbunden. Dies heißt, dass das Verschlussteil 5 mit seinem wenigstens einen elastischen Halteelement 6 und der Katheter 2 ursprünglich zusammenhängend gebildet sind und danach in die in Fig. 5 gezeigte Stellung gebracht werden. Auch hier ist das wenigstens eine elastische Halteelement 6 mit dem Katheter 2 elastisch verbunden, wie mittels einer Klebverbindung, so dass es das Verschlussteil 5 in der Katheterlängsrichtung von dem Katheter 2 elastisch abzuheben gestattet.

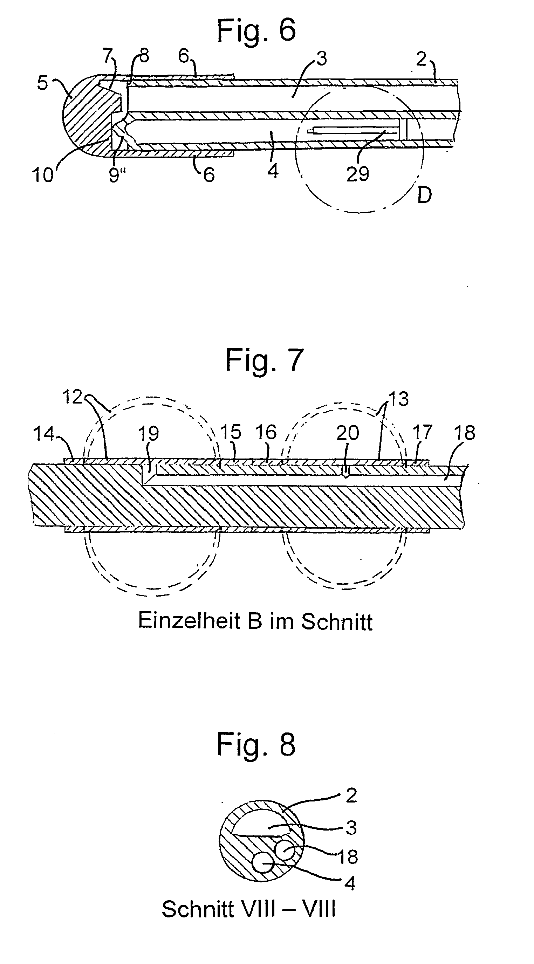

Fig. 6 veranschaulicht in einer vergrößerten Schnittansicht ausgehend von der in Fig. 3 dargestellten vergrößerten Schnittansicht die Verhältnisse, die vorliegen, wenn im Betätigungskanal 4 ein Betätigungsfluid für eine Betätigung des Verschlussteiles 5 und damit zur Öffnung des Harn-Entleerungskanals 3 wirksam wird. Im Unterschied zu den in Fig. 3 dargestellten Verhältnissen ist gemäß Fig. 6 die proximale Verschlusswand 9 durch eine Auswölbung bzw. Erhöhung 9" des Betätigungskanals 4 infolge eines erhöhten Betätigungsdrucks aus ihrer ursprünglichen, in Fig. 3 dargestellten Verschlussstellung in axialer Richtung des betreffenden Betätigungskanals 4 derart herausgedrückt, dass der Harn-Entleerungskanal 3 für einen ungehinderten Harndurchtritt geöffnet ist. In diesem Falle ist das konisch ausgebildete Anlageteil 7 mit der Anlagefläche des Verschlussteiles 5 von der End- bzw. Abschlusskante 8 am proximalen Katheterende abgehoben, wie dies in Fig. 6 veranschaulicht ist.

Nachdem zuvor die Verhältnisse näher betrachtet worden sind, die am proximalen Endabschnitt des in Fig. 1 dargestellten Katheters 2 vorliegen bzw. dort alternativ möglich sind, werden nachstehend die im Bereich zwischen dem betreffenden proximalen Endabschnitt des Katheters 2 und dessen distalen Endabschnitt vorliegenden Verhältnisse näher betrachtet.

In dem Bereich zwischen den proximalen und distalen Endabschnitten weist der Katheter 2 zur Abdichtung der Harnblase und zur Halterung des Katheters 2 im Blasenlumen eine mit einem Fluid auffüllbare Ballonanordnung auf, die im vorliegenden Fall aus zwei unmittelbar hintereinander angeordneten Ballons 12 und 13 besteht, die hier jeweils aus Silikon bestehen. Der Ballon 12 weist an seinen in Katheterlängsrichtung liegenden Enden jeweils ein Befestigungsteil 14, 15 auf, mit dem der betreffende Ballon 12 an dem Katheter 2 befestigt ist, wie durch eine biokompatible Klebverbindung. Der Ballon 13 weist in entsprechender Weise in Längsrichtung des Katheters 2 vorn bzw. hinten liegende Befestigungsteile 16, 17 auf, mit denen der betreffende Ballon 13 an dem Katheter 2 befestigt ist, wie durch eine biokompatible Klebverbindung. In Fig. 1 sind die beiden Ballons 12, 13 durch voll ausgezogene Linien im nicht gefüllten Zustand veranschaulicht; durch gestrichelte Linien ist in Fig. 1 der Füllzustand der Ballons 12, 13 angedeutet. Zwischen den Befestigungsteilen 14, 15 sowie 16, 17 sind somit die Aufblasbereiche der Ballons 12, 13 festgelegt. An dieser Stelle sei angemerkt, dass die beiden Ballons 12, 13 in Abweichung von den zuvor erläuterten Verhältnissen nicht unmittelbar nebeneinander liegend an dem Katheter 2 befestigt zu sein brauchen; vielmehr können sie auch in einem gewissen Abstand voneinander am Katheter 2 befestigt sein, wie dies beispielsweise in Fig. 1 der DE 196 21 420 C2 veranschaulicht ist.

Um den Aufbau des vorstehend kurz betrachteten Bereiches mit den Ballons 12, 13 des Katheters 2 näher zu veranschaulichen, wird auf Fig. 7 Bezug genommen, in der die in Fig. 1 mit B angedeutete Einzelheit in einer vergrößerten Schnittansicht dargestellt ist. Die beiden Ballons 12, 13 sind in Fig. 7 durch voll ausgezogene Linien ebenfalls im nicht befüllten Zustand dargestellt; der Füllzustand der beiden Ballons 12, 13 ist durch gestrichelte Linien angedeutet. Dabei geht aus Fig. 7 klar hervor, dass zwischen den Befestigungsteilen 14, 15 bzw. 16, 17 der beiden Ballons 12, 13 deren jeweiliger Aufblasbereich festgelegt ist. Die Füllung des Ballons 12 mit einem Fluid, wie beispielsweise mit Wasser oder einer Kochsalzlösung, erfolgt durch einen längs der Katheterwand verlaufenden und am distalen Endabschnitt des Katheters 2 verschlossenen Befüllungskanal 18, der im Bereich des Ballons 12, also des am proximalen Ende des Katheters 2 liegenden Ballons eine Austrittsöffnung 19 mit einem relativ großen Durchmesser aufweist. Durch diese Austrittsöffnung 19, die beispielsweise den Querschnitt des Befüllungskanals 18 aufweisen kann, ist das erwähnte Fluid zum Zwecke des Befüllens des Ballons 12 abgebbar. Um den zweiten Ballon 13 mit dem Fluid füllen zu können, ist hier der Ballon 13 mittels einer weiteren Austrittsöffnung 20 mit dem Befüllungskanal 18 verbunden. Diese Austrittsöffnung 20 weist einen geringeren Querschnitt auf als die Austrittsöffnung 19 im Bereich des Ballons 12. Durch diese Maßnahmen füllt das Fluid den Befüllungs- bzw. Aufblasbereich des Ballons 12 relativ schnell, und der Ballon 13 füllt sich langsamer als der Ballon 12. Der zuvor erwähnte Kanal 18 wird nun nicht nur zum Befüllen der Ballons 12, 13 mit einem Fluid genutzt, sondern er dient auch zur Ableitung des betreffenden Fluids bei Entleerung der Ballons 12, 13.

An dieser Stelle sei noch angemerkt, dass die beiden Ballons 12, 13 in dem Fall, dass der Befüllungskanal 18 lediglich mit dem Ballon 12 verbunden wäre, auch durch einen gesonderten Befüllungskanal gewissermaßen fluidmäßig in Reihe angeordnet sein könnten. Auch in diesem Falle würde zunächst der Ballon 12 befüllt werden und danach der Ballon 13.

Fig. 8 zeigt eine Schnittansicht längs der in Fig. 1 eingetragenen Schnittlinie VIII-VIII, das heißt im Bereich zwischen den beiden zuvor erwähnten Ballons 12, 13. Wie ersichtlich, weist der Katheter 2 in diesem Bereich den Harn-Entleerungskanal 3, den Betätigungskanal 4 und den an Hand von Fig. 7 erläuterten Kanal 18 auf.

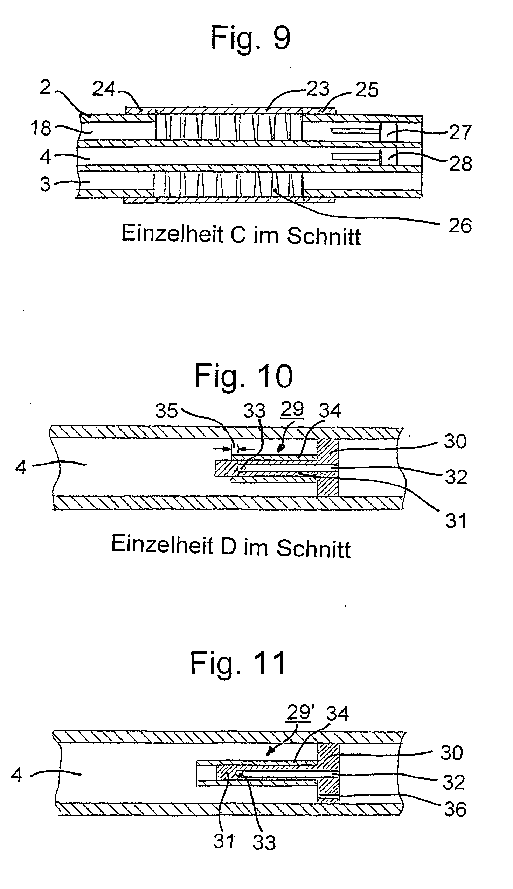

Zurückkommend auf Fig. 1 sei nunmehr noch eine Einzelheit C am distalen Endbereich des Katheters 2 näher betrachtet, die durch eine Strichpunktlinie umgeben ist. Wie dort angedeutet, befindet sich an diesem distalen Katheterendbereich ein Betätigungsballon 23, der hier ebenfalls aus Silikon besteht und der mit Befestigungsteilen 24, 25 am Katheter 2 befestigt ist, wie durch eine biokompatible Klebverbindung. Der nähere Aufbau des den Betätigungsballon 23 umfassenden Bereichs (Einzelheit C), wie er in Fig. 1 durch einen Strichpunktlinien-Kreis festgelegt ist, ist in einer vergrößerten Schnittansicht in Fig. 9 dargestellt.

Wie aus Fig. 9 ersichtlich ist, weist der Katheter 2 an seinem distalen Endbereich den nunmehr unten liegend dargestellten Harn-Entleerungskanal 3 und darüber liegend den Betätigungskanal 4 sowie den Befüllungskanal 18 auf. Der Betätigungskanal 4 und der Befüllungskanal 18 sind am in Fig. 9 rechts dargestellten distalen Ende des Katheters 2 durch Ventile 27 bzw. 28 verschließbar. Der Harn-Entleerungskanal 3 ist hingegen an dem betreffenden Katheterende offen. Die betreffenden Ventile 27, 28 sind zum Zwecke des Befüllens des Betätigungskanals 4 und des Befüllungskanals 18 mit entsprechenden Fluids zu öffnen, und sie sind außerdem zum Zwecke des Entleerens der betreffenden Kanäle 4, 18 zu öffnen, worauf hier jedoch nicht weiter näher eingegangen wird.

Der Betätigungsballon 23 ist, wie aus Fig. 9 ersichtlich ist, mit seinen Befestigungsteilen 24, 25 an gegenüberliegenden Bereichen des Katheters 2 befestigt. Er weist auf seiner Innenseite eine Vielzahl von Betätigungsnoppen 26 auf, mit denen ein gewisser mechanischer Druck auf die Wandung des Betätigungskanals 4 ausübbar ist. Nachdem die gesamte Vorrichtung flexibel ausgebildet ist, lässt sich die Wandung des Betätigungskanals 4 durch die erwähnten Noppen 26 auf eine mechanische Betätigung des Betätigungsballons 23 hin zusammendrücken, wodurch ein entsprechender Druck auf das im Betätigungskanal 4 befindliche Betätigungsfluid ausgeübt wird. Diese Druckausübung wird dann zur Öffnung des am proximalen Katheterende vorgesehenen Ventils ausgenutzt, wie dies in einer vergrößerten Schnittansicht gemäß Fig. 6 des betreffenden proximalen Katheterendes näher veranschaulicht ist. Wie im Zusammenhang mit Fig. 6 bereits erläutert, wird als Folge einer Druckausübung auf das Betätigungsfluid im Betätigungskanal 4 die an dessen proximalen Endbereich vorgesehene Verschlusswand 9 aus ihrer geraden Lage herausgeführt, so dass sich eine Erhöhung 9" ausbildet, die gegen die Betätigungsfläche bzw. Verschlusswand 10 des Verschlussteiles 5 wirkt. Dadurch wird das konisch ausgebildete Anlageteil 7 des Verschlussteiles 5 von der End- bzw. Abschlusskante 8 des Harn-Entleerungskanals 3 weggeführt, so dass der Harn-Entleerungskanal 3 für eine Harnentleerung freigegeben ist. Nach Druckentlastung in dem Betätigungskanal 4 kehrt die Verschlusswand 9 dieses Betätigungskanals 4 wieder in die in Fig. 3 bzw. 5 gezeigte Stellung zurück, in der der Harn-Entleerungskanal 3 wieder verschlossen ist.

Nunmehr sei noch auf eine in Fig. 6 durch einen Strichpunktlinien-Kreis markierte Einzelheit D im Betätigungskanal 4 eingegangen. Dazu wird insbesondere auf Fig. 10 Bezug genommen, die diese Einzelheit D in einer vergrößerten Schnittansicht zeigt. In dem Betätigungskanal 4 ist nahe des proximalen Katheterendes ein gesondertes Ventil 29 angeordnet, durch das in dem in Fig. 6 und 10 jeweils links dargestellten Bereich des Betätigungskanals 4 ein abgetrenntes Drucklumen gebildet ist, auf dessen Befüllen mit dem Betätigungsfluid durch Betätigen des in Fig. 1 und 6 dargestellten Betätigungsballons 23 das Verschlussteil 5 aus seiner Dichtungsanlage dem genannten Harn-Entleerungskanal 3 des Katheters 2 herausführbar ist. Das betreffende Ventil 29 weist ein mit dem Innenraum des Betätigungskanals 4, beispielsweise durch eine biokompatible Klebverbindung verbundenes Tragteil 30 und einen mit diesem Tragteil 30 verbundenen Ventilschaft 31 auf. Ferner weist das Ventil 29 eine vom Tragteil 30 her in den Ventilschaft 31 verlaufende Längsöffnung 32 und ein an deren proximalen Ende vorgesehenes Querloch 33 auf.

Der das Querloch 33 enthaltende Bereich des Ventilschafts 31 ist von einem Ventilschlauch 34 umgeben. Sowohl das Tragteil 30, wie der Ventilschaft 31 wie auch der Ventilschlauch 34 können vorzugsweise aus Silikon bestehen. In seinem Aufbau ähnelt das vorstehend betrachtete Ventil 29 somit einem herkömmlichen Fahrradschlauchventil.

Durch Festlegung des in Fig. 10 mit 35 bezeichneten Überstands des Ventilschlauchs 34 über dem Querloch 33 lässt sich die Stärke der Rückströmung aus dem in den in Fig. 6 und 10 links dargestellten Drucklumen des Betätigungskanals 4 in den in den betreffenden Figuren rechts dargestellten Betätigungskanalbereich festlegen. Dadurch ermöglicht das Ventil 29 ein Einströmen des Betätigungsfluids in den in Fig. 6 und 10 links dargestellten Bereich bzw. in das dortige Drucklumen des Betätigungskanals 4 mit einer ersten, relativ hohen Geschwindigkeit, und es lässt eine Rückströmung des betreffenden Betätigungsfluids aus dem betreffenden Bereich bzw. Drucklumen mit einer demgegenüber wesentlich geringeren zweiten Geschwindigkeit zu. Durch wiederholtes Betätigen des Betätigungsballons 23 am distalen Katheterende - was praktisch eine Pumpfähigkeit darstellt - lässt sich somit der in Fig. 6 und 10 links dargestellte Bereich (Drucklumen) des Betätigungskanals 4 mit dem Betätigungsfluid gewissermaßen aufpumpen. Danach entleert sich der betreffende Bereich (Drucklumen) des Betätigungskanals 4 wieder langsam, indem das Betätigungsfluid durch den Bereich zwischen Ventilschaft 31 und Ventilschlauch 34 wieder in den in Fig. 6 und 10 rechts dargestellten Bereich des Betätigungskanals 4 entweicht.

Nunmehr sei noch kurz auf Fig. 11 eingegangen, die eine alternative Realisierung des in Fig. 10 gezeigten Ventils 29 veranschaulicht. Das in Fig. 11 dargestellte Ventil ist mit 29' bezeichnet; es weist wie das in Fig. 10 dargestellte Ventil 29 ein Tragteil 30, einen Ventilschaft 31, eine Längsöffnung 32 und ein an deren proximalen Ende vorhandenes Querloch 33 sowie einen Ventilschlauch 34 auf, der hier allerdings weit über den Ventilschaft 31 hinaus ragt. Sämtliche Elemente des Ventils 29' können vorzugsweise aus Silikon bestehen. Im Unterschied zu dem in Fig. 10 dargestellten Ventil 29 weist das Ventil 29' in seinem Tragteil eine Rückströmungsöffnung 36 auf, die einen wesentlich kleineren Querschnitt besitzt als das Querloch 33. Besitzt das Querloch beispielsweise einen Durchmesser von 0,5mm, so liegt der Durchmesser der Rückströmungsöffnung 35 im Mikrometerbereich, wie im Bereich von beispielsweise 10µm bis 250µm.

### Bezugszeichenliste

| | |
|---|---|
| 1 | Vorrichtung |
| 2 | Katheter |
| 3 | Harn-Entleerungskanal, Entleerungskanal |
| 4 | Betätigungskanal |
| 5 | Verschlussteil |
| 6 | Halteelement |
| 7 | Anlageteil |
| 7' | Stellung des Anlageteiles |
| 8 | Endkante, Abschlusskante |
| 9 | Verschlusswand |
| 9" | Erhöhung, Auswölbung |
| 10 | Betätigungsfläche |
| 11 | Gelenkelement |
| 12 | Ballon |
| 13 | Ballon |
| 14, 15 | Befestigungsteil |
| 16, 17 | Befestigungsteil |
| 18 | Befüllungskanal |
| 19 | Austrittsöffnung |
| 20 | Austrittsöffnung |
| 23 | Betätigungsballon |
| 24, 25 | Befestigungsteil |
| 26 | Betätigungsnoppen |
| 27, 28 | Ventil |
| 29, 29' | Ventil |
| 30 | Tragteil |
| 31 | Ventilschaft |
| 32 | Längsöffnung |
| 33 | Querloch |
| 34 | Ventilschlauch |
| 35 | Überstand |
| 36 | Rückströmungsöffnung |
| A, B, C, D | Einzelheit |
| II-II | Schnitt |
| VIII-VIII | Schnitt |

## Patentansprüche

1. Vorrichtung (1) zur Behandlung von Hamblasen-Entleerungsstörungen eines Menschen, mit einem in die Harnröhre einführbaren Katheter (2), der einen Harn-Entleerungskanal (3) aufweist und der zur Abdichtung der Harnblase und zur Halterung des Katheters (2) im Blasenlumen eine mit einem Fluid auffüllbare Ballonanordnung (12, 13) trägt, die durch mindestens einen längs der Katheterwand verlaufenden und am distalen Endabschnitt des Katheters (2) verschlossenen Kanal (18) mit dem genannten Fluid auffüllbar bzw. entleerbar ist, und mit einem in einem proximalen Endabschnitt des Katheters (2) untergebrachten, selbsttätig schließenden Ventil (7, 8), wobei die Länge des Katheters (2) derart bemessen ist, dass dessen distales Ende im eingesetzten Zustand innerhalb der Harnröhre liegt und der betreffende proximale Endabschnitt einen hydraulischen Betätigungsmechanismus zum Öffnen des Ventils (7, 8) trägt, wobei der Betätigungsmechanismus durch mechanischen Druck auf einen am distalen Endabschnitt des Katheters (2) angeordneten, mit Betätigungsfluid gefüllten und über einen Verbindungskanal (4) mit dem Betätigungsmechanismus verbundenen Betätigungsballon (23) hydraulisch beaufschlagbar ist, wobei der genannte Betätigungsmechanismus **dadurch** gebildet ist, dass das Ventil (7, 8) ein mit dem genannten proximalen Endabschnitt des Katheters (2) derart elastisch verbundenes Verschlussteil (5) enthält, dass dieses im betätigungslosen Zustand des Betätigungsballons (23) den Katheter (2) an dem genannten proximalen Endabschnitt abdichtet, **dadurch gekennzeichnet dass** das betreffende Verschlussteil (5) allein auf eine hydraulische Beaufschlagung des Verbindungskanals (4) infolge des durch Betätigen des Betätigungsballons (23) **dadurch** an einer einer Betätigungsfläche (10) des Verschlussteiles (5) gegenüberliegenden Verschlusswand (9) des Verbindungskanals (4) an dem genannten proximalen Endabschnitt wirksamen, die Verschlusswand (9) in axialer Richtung des Betätigungskanals (4) herausdrückenden hydraulischen Druckes aus der Dichtungsanlage soweit herausführbar ist, dass der Harn-Entleerungskanal (3) des Katheters (2) für einen ungehinderten Harndurchtritt geöffnet ist.

2. Vorrichtung nach Anspruch 1, **dadurch gekennzeichnet, dass** das Verschlussteil (5) mit einer konisch ausgebildeten Anlagefläche (7) den Harn-Entleerungskanal (3) des Katheters (2) an einer Abschlusskante (8) am proximalen Katheterende abzudichten gestattet.

3. Vorrichtung nach Anspruch 1 oder 2, **dadurch gekennzeichnet, dass** das Verschlussteil (5) und der proximale Endabschnitt des Katheters (2) mittels Halteelementen (6) elastisch miteinander verbunden sind.

4. Vorrichtung nach Anspruch 1 oder 2, **dadurch gekennzeichnet, dass** das Verschlussteil (5) mit dem proximalen Endabschnitt des Katheters (2) durch ein Gelenkelement (11) sowie mittels wenigstens eines elastischen Halteelements (6) verbunden ist.

5. Vorrichtung nach Anspruch 3 oder 4, **dadurch gekennzeichnet, dass** das jeweilige elastische Halteelement (6) an dem Verschlussteil (5) oder am proximalen Endabschnitt des Katheters (2) gebildet und mit seinem jeweils anderen Ende am proximalen Ende des Katheters (2) bzw. am Verschlussteil (5) gesondert befestigt ist.

6. Vorrichtung nach Anspruch 5, **dadurch gekennzeichnet, dass** das jeweilige Halteelement (6) mit seinem jeweils anderen Ende am proximalen Ende des Katheters (2) bzw. am Verschlussteil (5) durch eine Klebverbindung befestigt ist.

7. Vorrichtung nach einem der Ansprüche 1 bis 6,
**dadurch gekennzeichnet, dass** der mit dem Betätigungsballon (23) verbundene Verbindungs- bzw. Betätigungskanal (4) im Bereich des proximalen Endabschnitts des Katheters (2) ein durch ein gesondertes Ventil (29) abgetrenntes Drucklumen enthält, auf dessen Befüllen mit dem Betätigungsfluid durch Betätigen des genannten Betätigungsballons (23) das Verschlussteil (5) aus dessen Dichtungsanlage an dem genannten Harn-Entleerungskanal (3) des Katheters (2) abhebbar ist.

8. Vorrichtung nach Anspruch 7, **dadurch gekennzeichnet, dass** das genannte Ventil (29) so ausgebildet ist, dass es ein Einströmen des Betätigungsfluids in den genannten Drucklumen mit einer ersten, relativ hohen Geschwindigkeit ermöglicht und eine Rückströmung des betreffenden Betätigungsfluids aus dem genannten Drucklumen mit einer demgegenüber wesentlich geringeren zweiten Geschwindigkeit zulässt.

9. Vorrichtung nach Anspruch 8, **dadurch gekennzeichnet, dass** das genannte Ventil (29), welches das Drucklumen von dem Betätigungsballon (23) trennt, einen Ventilschaft (31) aufweist, der im Drucklumen eine vom Betätigungsballon (23) her zugängliche Durchgangsöffnung (32, 33) aufweist, die von einem Ventilschlauch (34) umgeben ist.

10. Vorrichtung nach einem der Ansprüche 1 bis 9,
**dadurch gekennzeichnet, dass** der Katheter (2) und das Verschlussteil (5) aus Silikon bestehen.

11. Vorrichtung nach einem der Ansprüche 1 bis 10,
**dadurch gekennzeichnet, dass** die Ballonanordnung (12, 13) und der Betätigungsballon (23) aus Silikon bestehen.

12. Vorrichtung nach einem der Ansprüche 1 bis 11,
**dadurch gekennzeichnet, dass** das Betätigungsfluid Öl ist.

13. Vorrichtung nach Anspruch 12, **dadurch**
**gekennzeichnet, dass** das Öl Olivenöl ist.

## Claims

1. Device (1) for treating bladder-emptying dysfunctions of a human, comprising
a catheter (2) that can be introduced into the urethra,
which catheter has a urine-emptying channel (3) and carries a balloon arrangement (12, 13) that can be filled with a fluid, to seal the bladder and to hold the catheter (2) in the bladder lumen,
which arrangement can be filled with or emptied of the said fluid by means of at least one channel (18) that runs along the catheter wall and is sealed off at the distal end segment of the catheter (2);
and an automatically closing valve (7, 8) accommodated in a proximal end segment of the catheter (2),
whereby the length of the catheter (2) is dimensioned so that its distal end lies within the urethra in the inserted state,
and the said proximal end segment carries a hydraulic activation mechanism for opening the valve (7, 8),
whereby said activation mechanism can be hydraulically impacted by means of mechanical pressure on an activation balloon (23) that is disposed on the distal end segment of the catheter (2), filled with activation fluid, and connected with the activation mechanism by way of a connection channel (4),
whereby the said activation mechanism is formed in that the valve (7, 8) contains a closure part (5) elastically connected with the said proximal end segment of the catheter (2) in such a manner that this part seals off the catheter (2) at the said proximal end segment in the non-activated state of the activation balloon (23),
**characterized in that** the said closure part (5) can be moved out off the sealing contact solely in response to a hydraulic impact by actuating the activation balloon (23) by means of the effective hydraulic pressure built up thereby at a closure wall (9) of said connection channel (4) at the said proximal end segment,
wherein the closure wall (9) of said connection channel (4) is opposite to an activation surface (10) of the said closure part (5) and is pressed out of the connection channel (4) in its axially direction by the said hydraulic pressure so that the urine-emptying channel (3) of the catheter (2) is opened by said closure part in an extent for unhindered passage of urine.

2. Device according to claim 1, **characterized in that** the valve closure part (5) having a conically shaped contact surface (7) that makes it possible to seal off the urine-emptying channel (3) of the catheter (2) at an end edge (8) at the proximal catheter end.

3. Device according to claim 1 or 2, **characterized in that** the closure part (5) and the proximal end segment of the catheter (2) are elastically connected with one another by means of holder elements (6).

4. Device according to claim 1 or 2, **characterized in that** the closure part (5) is connected with the proximal end segment of the catheter (2) by means of a joint element (11) as well as by means of at least one elastic holder element (6).

5. Device according to claim 3 or 4, **characterized in that** one end of each elastic holder element (6) is formed on the closure part (5) or on the proximal end segment of the catheter (2), and is separately attached to the proximal end of the catheter (2) or to the closure part (5), respectively, with its other end.

6. Device according to claim 5, **characterized in that** the other end of each holder element (6) is separately attached to the proximal end segment of the catheter (2) or to the closure part (5), respectively, by means of a glued connection.

7. Device according to one of the claims 1 to 6, **characterized in that** the connection or activation channel (4), respectively connected with the activation balloon (23) contains a pressure lumen separated by a separate valve (29) in the region of the proximal end segment of the catheter (2), and when this lumen is filled with the activation fluid, by means of activation of the said activation balloon (23), the closure part (5) can be lifted up from its sealing contact on the said urine-emptying channel (3) of the catheter (2).

8. Device according to claim 7, **characterized in that** the said valve (29) is configured in such a manner that it allows in-flow of the activation fluid into the said pressure lumen at a first, relatively high velocity, and permits back-flow of the activation fluid in question from the said pressure lumen at a significantly lower second velocity, in comparison.

9. Device according to claim 8, **characterized in that** the said valve (29), which separates the pressure lumen from the activation balloon (23), has a valve shaft (31) that has a passage opening (32, 33) in the pressure lumen that is accessible from the activation balloon (23), which opening is surrounded by a valve tube (34).

10. Device according to one of the claims 1 to 9, **characterized in that** the catheter (2) and the closure part (5) consist of silicone.

11. Device according to one of the claims 1 to 10, **characterized in that** the balloon arrangement (12, 13) and the activation balloon (23) consist of silicone.

12. Device according one of the claims 1 to 11, **characterized in that** the activation fluid is oil.

13. Device according to claim 12, **characterized in that** the oil is olive oil.

## Revendications

1. Dispositif (1) de traitement de troubles d'évacuation de la vessie de l'être humain, comportant un cathéter (2) pouvant être introduit dans l'urètre, qui présente un canal d'évacuation de l'urine (3) et qui, pour étanchéifier la vessie et pour maintenir le cathéter (2) dans le lumen de la vessie, supporte un dispositif de ballon (12, 13) pouvant être rempli de fluide qui peut être rempli ou vidé dudit fluide par au moins un canal (18) s'étendant le long de la paroi du cathéter et fermé au niveau de la section terminale distale du cathéter (2), et une soupape (7, 8) se fermant automatiquement logée dans une section terminale proximale du cathéter (2), la longueur du cathéter (2) étant dimensionnée de manière à ce que son extrémité distale, dans l'état posé, se situe à l'intérieur de l'urètre et la section terminale proximale en question porte un mécanisme d'actionnement hydraulique permettant d'ouvrir la soupape (7, 8), le mécanisme d'actionnement pouvant être sollicité par système hydraulique par une pression mécanique appliquée sur un ballon d'actionnement (23) disposé au niveau de la section terminale distale du cathéter (2), rempli de fluide d'actionnement et relié par un canal de liaison (4) au mécanisme d'actionnement,
ledit mécanisme d'actionnement étant réalisé de telle sorte que la soupape (7, 8) contienne une partie de fermeture (5) reliée élastiquement à ladite section terminale proximale du cathéter (2) de telle sorte que ladite partie de fermeture (5) étanchéifie dans l'état non actionné du ballon d'actionnement (23) le cathéter (2) au niveau de ladite section terminale proximale,
**caractérisé en ce que**
ladite partie de fermeture (5) peut être ressortie par une simple sollicitation hydraulique du canal de liaison (4) hors de l'installation d'étanchéité, sous l'effet de la pression hydraulique repoussant la paroi de fermeture (9) dans la direction axiale du canal d'actionnement (4), agissant par l'actionnement du ballon d'actionnement (23) de ce fait sur une paroi de fermeture (9) du canal de liaison (4) opposée à une surface d'actionnement (10) de la partie de fermeture (5), au niveau de ladite section terminale proximale, dans une mesure telle que le canal d'évacuation de l'urine (3) du cathéter (2) soit ouvert pour permettre un libre passage de l'urine.

2. Dispositif selon la revendication 1, **caractérisé en ce que** la partie de fermeture (5) est pourvue d'une surface d'appui (7) sous forme conique, pour étancher le canal d'évacuation de l'urine (3) du cathéter (2) au niveau d'un bord de terminaison (8) à l'extrémité proximale du cathéter.

3. Dispositif selon la revendication 1 ou 2, **caractérisé en ce que** la partie de fermeture (5) et la section terminale proximale du cathéter (2) sont reliées élastiquement l'une à l'autre au moyen d'éléments de retenue (6).

4. Dispositif selon la revendication 1 ou 2, **caractérisé en ce que** la partie de fermeture (5) est reliée à la section terminale proximale du cathéter (2) par un élément articulé (11) ainsi qu'au moyen d'au moins un élément de retenue élastique (6).

5. Dispositif selon la revendication 3 ou 4, **caractérisé en ce que** l'élément de retenue élastique respectif (6) est formé sur la partie de fermeture (5) ou sur la section terminale proximale du cathéter (2) et est fixé séparément par son autre extrémité respective à l'extrémité proximale du cathéter (2) ou à la partie de fermeture (5).

6. Dispositif selon la revendication 5, **caractérisé en ce que** l'élément de retenue respectif (6) est fixé par son autre extrémité respective à l'extrémité proximale du cathéter (2) ou à la partie de fermeture (5) par une liaison adhésive.

7. Dispositif selon l'une quelconque des revendications 1 à 6, **caractérisé en ce que** le canal de liaison ou d'actionnement (4) relié au ballon d'actionnement (23) contient, dans la région de la section d'extrémité proximale du cathéter (2), un lumen de pression séparé par une soupape séparée (29), qui, lorsqu'il est rempli avec le fluide d'actionnement par actionnement dudit ballon d'actionnement (23), entraîne le soulèvement de la partie de fermeture (5) hors de son appui étanche sur ledit canal d'évacuation d'urine (3) du cathéter (2).

8. Dispositif selon la revendication 7, **caractérisé en ce que** ladite soupape (29) est réalisée de telle sorte qu'elle permette un écoulement du fluide d'actionnement dans ledit lumen de pression avec une première vitesse relativement élevée, et qu'elle autorise un reflux dudit fluide d'actionnement hors dudit lumen de pression avec une deuxième vitesse considérablement plus faible par comparaison.

9. Dispositif selon la revendication 8, **caractérisé en ce que** ladite soupape (29), qui sépare le lumen de pression du ballon d'actionnement (23), présente une tige de soupape (31) qui présente dans le lumen de pression une ouverture de passage (32, 33) accessible depuis le ballon d'actionnement (23), qui est entourée par un tuyau de soupape (34).

10. Dispositif selon l'une quelconque des revendications 1 à 9, **caractérisé en ce que** le cathéter (2) et la partie de fermeture (5) se composent de silicone.

11. Dispositif selon l'une quelconque des revendications 1 à 10, **caractérisé en ce que** le dispositif de ballon (12, 13) et le ballon d'actionnement (23) se composent de silicone.

12. Dispositif selon l'une quelconque des revendications 1 à 11, **caractérisé en ce que** le fluide d'actionnement est de l'huile.

13. Dispositif selon la revendication 12, **caractérisé en ce que** l'huile est de l'huile d'olive.
